# EUROPEAN PATENT APPLICATION

(11) **EP 0 930 363 A2**
(43) Date of publication of application: **21.07.1999**
(21) Application number: 99100822.8
(22) Date of filing: 18.01.1999
(51) Int. Cl.: C12N 11/00, C12P 1/00, C12S 13/00, C12Q 1/24, C12N 15/63, C12N 5/10, C12N 1/19, C12N 1/21, C12N 9/02, C12N 15/53, C12M 1/40

(54) **Immobilization of cohesive or adhesive cells and use thereof**

(30) Priority: 19.01.1998 JP 2285898
(71) Applicant: KANSAI CHEMICAL ENGINEERING CO. LTD, Amagasaki-shi Hyogo 660-0053 (JP)
(72) Inventor: Fukuda, Hideki, Kobe-city, Hyogo, 655-0871 (JP)
(74) Representative: Bühling, Gerhard, Dipl.-Chem.

(57) **Abstract**

The present invention provides an immobilized cell aggregate. A cell with lowered cell activity or dead cell in the immobilized cell aggregate can drop out from the immobilized cell aggregate due to loss of cohesive or adhesive activity. Therefore, only living cells can remain in the immobilized cell aggregate. By using the immobilized cell aggregate, a desired material can be efficiently produced and environmental pollutants can be efficiently decomposed. The cohesiveness or adhesiveness can be obtained by incorporating genes. Moreover, by introducing genes for secretion and production for a desired material into cells of immobilized cell aggregate, the desired material can be efficiently produced.

## Description

### BACKGROUND OF THE INVENTION

### 1.FIELD OF THE INVENTION:

The present invention relates to immobilization of cohesive cells or adhesive cells and use thereof. More precisely, the present invention relates to an immobilized cell aggregate comprising a support and cohesive cells or adhesive cells. A cell in the aggregate can grow in the aggregate and a cell with lowered cell activity or a dead cell can drop out from the aggregate due to loss of cohesive or adhesive activity. The present invention also relates to a method of manufacturing useful materials by use of the immobilized cell aggregate.

### 2.DESCRIPTION OF THE RELATED ART:

Many studies have been performed to produce useful materials or decompose environmental pollutants by use of immobilized cells. However, there is a problem that although the activity of an immobilized cell becomes lower or the immobilized cell may die, such cells will still remain in the immobilized cell. Therefore, there remains the problem that the productivity of the desired materials or the decomposition activity of the environmental pollutant becomes low.

### SUMMARY OF THE INVENTION

The present invention has solved the above-mentioned problems by using an immobilized cell aggregate composed of a support and cohesive or adhesive cells.

According to the present invention, since a cell with lowered cell activity or a dead cell can drop out from the immobilized cell aggregate due to loss of cohesive or adhesive activity, only living cells can remain in the immobilized cell aggregate. Therefore, by using the immobilized cell aggregate of the present invention, a desired material can be efficiently produced and environmental pollutants can be efficiently decomposed.

The present invention relates to an immobilized cell aggregate comprising a support and cohesive or adhesive cells,
wherein a cohesive or adhesive cell can be immobilized on the support;
wherein the cohesive or adhesive cell is capable of growth in the aggregate; and
wherein a cell with lowered cell activity or a dead cell can drop out from the aggregate due to loss of its cohesive or adhesive activity.

In a preferred embodiment, the cell is a non-cohesive or non-adhesive cell into which genes for cohesiveness or adhesiveness are introduced.

In a preferred embodiment, the cell is cohesive yeast, a cohesive bacterium or an animal cell.

In a preferred embodiment, the cell is cohesive yeast or non-cohesive yeast into which genes for cohesiveness are introduced.

In a preferred embodiment, the support is a porous support.

In a preferred embodiment, the aggregate is packed in a column.

Another invention is related to a method for producing a desired material comprising the steps of subjecting an immobilized cell aggregate to cultivation;
wherein a cohesive or adhesive cell can be immobilized on the support;
wherein the cohesive or adhesive cell is capable of growth in the aggregate and can produce the desired material; and
wherein a cell with lowered cell activity or a dead cell can drop out from the aggregate due to loss of its cohesive or adhesive activity.

In a preferred embodiment, the cell is a non-cohesive or non-adhesive cell into which genes for cohesiveness or adhesiveness are introduced.

In a preferred embodiment, the desired material is secreted from the cell.

In a preferred embodiment, the desired material is secreted from the cell into which genes for secretion and production of the desired material are introduced.

In a preferred embodiment, the cell is cohesive yeast, a cohesive bacterium or an animal cell.

In a preferred embodiment, the cell is cohesive yeast or non-cohesive yeast into which genes for cohesiveness are introduced.

In a preferred embodiment, the support is a porous support.

In a preferred embodiment, the aggregate is packed in a column.

The present invention also relates to a method for selecting a cohesive or adhesive cell that can: attach to a support so as to form an immobilized cell aggregate; grow in the immobilized cell aggregate; produce a desired material; and drop out from the immobilized cell aggregate due to lowered cell activity or death;
wherein the method comprises:
introducing genes for cohesiveness or adhesiveness and/or genes for producing the desired material into a cell so as to obtain a recombinant cell;
cultivating the recombinant cell with the support so as to form an immobilized cell aggregate; detecting and/or measuring the desired material that is produced from the immobilized cell aggregate; and
recovering the cohesive or adhesive cell.

Another invention relates to a method for preparing an immobilized cell aggregate comprising a support and cohesive or adhesive cells, wherein a cohesive or adhesive cell is capable of growth in the aggregate and wherein a cell with lowered cell activity or a dead cell can drop out from the aggregate due to loss of its cohesive or adhesive activity;
wherein the method comprises:
introducing genes for cohesiveness or adhesiveness and/or genes for producing the desired material into a cell so as to obtain a recombinant cell;
cultivating the recombinant cell with the support so as to form an immobilized cell aggregate;
recovering the immobilized cell aggregate.

Yet another invention relates to an expression cassette which comprises genes for cohesiveness or adhesiveness and genes for regulating the production of a desired material,
wherein the cassette is expressed so as to convert a non-cohesive or non-adhesive cell into a cohesive or adhesive cell; the cohesive or adhesive cell being immobilized on a support so as to form an immobilized cell aggregate; the cohesive or adhesive cell being capable of growth in the aggregate; a cell of lowered cell activity or a dead cell being able to drop out from the support due to loss of cohesive or adhesive activity; and
Wherein the cassette is expressed so as to produce the desired material.

In a preferred embodiment, the cassette has genes for cohesiveness of yeast and a promoter sequence for regulating the expression of the gene of the yeast for the desired material.

### BRIEF DESCRIPTION OF THE DRAWINGS

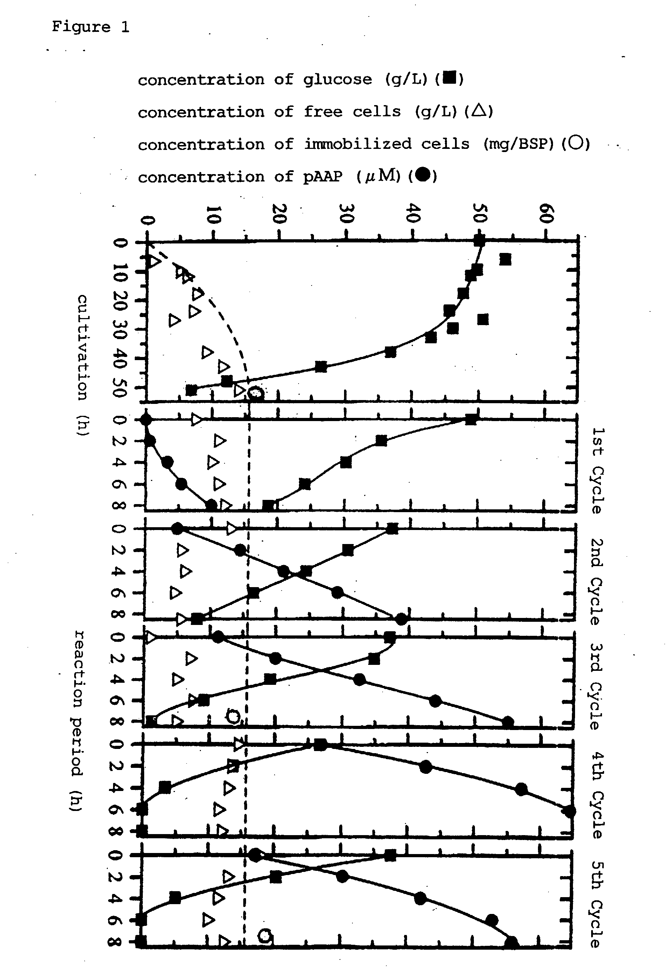

Figure 1 shows activities in each cycle of reaction in a repeated use of an immobilized cell aggregate including adhesive yeast.

### DESCRIPTION OF THE EMBODIMENTS

An immobilized cell aggregate including cohesive or adhesive cells and the preparation method of the immobilized cell aggregate of the present invention will be described.

The word "immobilized" used in the present specification means that cells are not free. For example, cells are bound or attached to a support or trapped in the support.

The phrase "capable of growth" used in the present specification means that cells can increase their number although the cells are immobilized on the support. This also means that cells in the immobilized cell aggregate can maintain their number although cells with lowered cell activity or dead cells can drop out from the immobilized cell aggregate.

The word "cohesive" used in the present specification means a property of a cell (such as bacteria, yeast and so on) in that when the cells are suspended or dispersed in a liquid, the cells can bind to each other to form a cell aggregate.

The word "adhesive" used in the present specification means a property of a cell that can attach or bind to others to form a cell aggregate.

The phrase "an immobilized cell aggregate" used in the present specification means an assembly of cohesive or adhesive cells in or on a support.

The phrase "cells with lowered cell activity" means that the activity of a cell is weakened although the cell is not dead, or a cell becomes unable to cohere or adhere to others. Examples of lowered activity are: decrease of cohesive or adhesive activity due to loss of enzymes for cohesiveness or adhesiveness; or, lowered activity of replication or translation of DNA(s) coding for enzyme(s) regarding the cohesive or adhesive activity.

Cells used in the present invention are, for example, yeast, bacteria, fungi (including mold), plant cells and animal cells. In view of handling operation and safety, yeast, bacteria and animal cells are preferred.

Further, cells used in the present invention may be cells that are given cohesive or adhesive activity by introducing genes for cohesiveness or adhesiveness into non-cohesive or non-adhesive cells.

The phrase "genes for cohesiveness or adhesiveness" includes a structural gene coding for a substance relating cohesive or adhesive activity, for example, chitin or lectin of yeast and fibronectin of an animal cell. The word also means a set of genes comprising a structural gene and regulatory genes which regulate expression of the structural gene, including operators, promoters, terminators, and enhancers.

As genes for cohesive activity, *FLO1* (J. Watari et al. Agric. Biol. Chem. 55:1547(1991); G. G. Stewart et al., Can. J. Microbiol.23: 441 (1977); I. Russell et al., Inst. Brew., 86: 120 (1980); C. W. Lewis et al. J. Inst. Brew. 82:158 (1976)), *FLO5* (I. Russell et al. J. Inst. Brew. 85: 95 (1979)), and *FLO8* (I. Yamashita et al. Agric. Biol. Chem. 48: 131(1984)) are exemplified.

The phrase "introduction of genes" means that genes are introduced and expressed into a cell using any suitable method to introduce and express the genes. Examples of suitable methods to introduce genes into a cell are transformation, transduction, transfection, co-transfection, and electroporation. As a host cell, bacteria, yeast, fungi, or plant or animal cells can be used. In a transformed cell, the introduced genes exist as a form of plasmid, or as a form of insert or homologous recombinant in a gene of a host cell so that a phenotype is expressed.

For the immobilization of cohesive or adhesive cells on a support, any suitable method including a support binding method, cross-linking method or entrapping method can be applied. Among them, the support binding method is the most suitable. The support binding method includes chemical adsorption (cells are adsorbed to an ion-exchange resin) and physical adsorption. In the present invention, the physical adsorption method using a porous support is most suitable in view of handling the cohesive or adhesive cell and allowing cells with lowered cell activity and dead cells to dropout.

The word "support" used in the present invention means a material which can immobilize cells and which is water-insoluble or insoluble to a specific solvent. As a support, synthetic resin is preferable. Among the resins, solid or resin foam such as polyvinylalcohol, polyurethane foam, polystyrene foam, polyacrylamide, porous resin of polyvinyl formal, silicone foam, or porous cellulose is preferably used. In considering the growth of cells or the dropping out of the cells with lowered cell activity or dead cells, a porous support is preferable. A certain size of the openings in the porous support is required so that cells can enter into the support and grow in the support. Thus, the size of the opening depends upon the cell size, and the size is preferably from about 10 µm to about 500 µm for bacteria, from about 50 µm to about 1,000 µm for yeast, fungi and plant cells, and from about 30 µm to about 250 µm for animal cells.

The shape of the support is not limited; however, in view of the strength of the support and efficiency of the culture, a spherical or cubic shape is preferable. In the case of a spherical shape, a diameter from about 2mm to 50mm is preferable. In the case of a cube, a side about 2mm to 50mm is preferable.

The cells used in the present invention can be immobilized onto the support so as to form an immobilized cell aggregate and grow on an immobilized cell aggregate. The immobilized cell aggregate of the present invention has the characteristic feature that the cells with lowered cell activity or dead cells can easily drop out from the immobilized cell aggregate. In considering the above, it is most preferable to immobilize the cells to the support by use of physical adsorption. No special technique is necessary for physical adsorption. By merely culturing a cohesive or adhesive cell with the porous support, the cell will enter into an opening pore of the porous support and adsorb to the support. Thus, an immobilized cell aggregate comprised of the support and the cohesive or adhesive cell that is immobilized on the support and capable of growth can be obtained.

The thus-obtained immobilized cell aggregate can be used as a bio-reactor by suspending or being packed in the column. Since cells with lowered cell activity or dead cells can be dropped out from the immobilized cell aggregate, the activity of the immobilized cell aggregate may not be decreased even by continuous cultivation or repeated use of the immobilized cell aggregate. Therefore, the immobilized cell aggregate can be used especially in producing a useful substance or decomposing a pollutant, efficiently.

Next, production of the useful substance using the immobilized cell aggregate comprised of the support and the cohesive or adhesive cell that is immobilized on the support and capable of growth on the support will be described.

A special method is not required for producing the desired useful substance. By merely culturing the immobilized cell aggregate, the desired product can be produced. Since the cohesive or adhesive cell used in the present invention may drop out from the immobilized cell aggregate if the activity of the cell becomes lower or if the cell has died, the immobilized cell aggregate may be composed of almost all living cells. Thus, high reactivity of the immobilized cell aggregate can be maintained.

As a desired product, a protein, amino acid, enzyme or antibody that is used for pharmaceuticals or food can be produced. As a desired product, an enzyme used for bio-conversion of a pollutant into, for example, a non-toxic substance can also be produced.

In order to produce the desired product, genes for producing the desired product may be introduced into the cohesive or adhesive cell. Alternately, in the case of using a non-cohesive or non-adhesive cell, genes for producing the desired product and genes for cohesiveness or adhesiveness may be introduced into the non-cohesive or non-adhesive cell. In addition to the genes above, genes for excreting the product can also be preferably introduced into the above cell so that the desired product can be secreted from the cell.

The phrase "genes for producing the desired product" used in the present specification includes a structural gene coding for the desired product and regulatory genes (sequence) such as operators, promoters, terminators or enhancers that regulate the expression of the structural gene.

An expression cassette used in the present invention can be expressed in a host cell so as to confer cohesiveness or adhesiveness to the non-cohesive or non-adhesive cell and also confer the productivity of the desired product to the host cell.

The phrase "expression cassette" used in the present specification includes a vector or a plasmid having cloning sites for genes for producing a desired product and, if necessary, genes for cohesiveness or adhesiveness. The expression cassette can exist independently as the form of a vector or plasmid in the host cell or as an integrated form in the genome of the host cell. Preferably, the expression cassette may include regulatory genes for the structural gene for the desired product so that if the desired structural gene is introduced into the suitable site of the cassette, the gene for the desired product can be expressed easily and efficiently. More preferably, the desired structural gene can be joined to a leader sequence so that the desired product can be secreted from the host cell. Even if a proper cloning site for joining the leader sequence and the structural gene is not present, ordinary skill in the art can join the leader sequence and the structural gene for the desired product correctly by using site specific mutagenesis. By employing genes having leader sequneces, the desired product can be secreted from the cell to the outside (including periplasm) of the cell. The phrase "genes for regulating the production of the desired product" includes regulatory sequences such as operators, promoters, terminators or enhancers that regulate the expression of the gene.

Genes used in the present invention and expression cassette can be prepared by using a suitable gene recombination technique. The obtained genes or the expression cassette can be introduced into the host cell in the same manner as described above so as to obtain a recombinant cell.

Whether or not the desired gene has been introduced can be determined by the following method.

If the host cell is a cohesive or adhesive cell, the recombinant cell is cultured in the presence of a porous support so as to form an immobilized cell aggregate, and then the production of the desired product can be detected.

If the host cell is a non-cohesive or non-adhesive cell, first, the recombinant cell is cultured in the presence of the porous support. If an immobilized cell aggregate can be formed, the recombinant cell is determined to have introduced the genes for cohesiveness or adhesiveness. Next, the recombinant cell can be tested for producing the desired product.

By using the above mentioned method, a cohesive or adhesive recombinant cell having introduced the gene for the desired substance (transformant) can be obtained. By using the tranformant, an immobilized cell aggregate can be obtained as described above.

The thus-obtained immobilized cell aggregate can be used for the production of the desired substance or the removal of the pollutant.

Hereinafter, the present invention will be described by way of example; however, the following example is not intended to limit the scope of the present invention.

### Example:

P450 mono-oxygenase of rat liver microsome catalyses an addition of monoatomic oxygen to a wide range of fat soluble materials in the presence of molecular oxygen. In this example, a gene for P450 mono-oxygenase of rat liver microsome is introduced into a cohesive yeast so as to obtain a transformed yeast. The conversion of acetanilide into acetaminophen using the transformed yeast can be performed.

### (Materials used in the experiments)

The yeast used in this example is cohesive Saccharomyces cerevisiae ATCC 60715. The plasmid pAMR2 (H.Murakami et al., J.Biochem. 108: 859 (1990)), which co-expresses reductase of Saccharomyces cerevisiae (Y.Yabusaki et al., J. Biochem. 103: 1004(1988)), and P450 of rat liver microsome (K. Oeda et al., DNA 4:203(1985)) was used as a plasmid.

The amount of expression of P450 in the transformed yeast was measured by the CO difference spectrum of the yeast cell mass. The amount of acetaminophen produced from acetanilide was measured by HPLC. The amount of ethanol production was measured by gas chromatography. Glucose was measured by mutarose and glucose oxidase. Each weight of free cells and imobilized cells was measured by the drying cell method.

### (Example 1:transformation of yeast and expression of P450)

The transformation of yeast was performed by using the lithium acetate method. The cohesive yeast Saccharomyces cerevisiae ATCC 60715 was used as a host and the plasmid pAMR2 was introduced into the yeast so as to obtain a transformant (hereinafter referred to as ATCC 60715 (pAMR2)).

Into a bubble column-type fermenter containing 3L of SD medium (0.67W/V% nitrogen base w/o amino acids, 5W/V% glucose, 160 µg/ml of histidine), dice of polyurethane foam with 6mm sides were addded so that about 1,000 dice per liter of the medium was obtained. To this fermenter, the transformant, ATCC60715 (pAMR2), was inoculated and cultivated at 30°C for 51 hours so as to immobilize the transformant to the polyurethane foam and thus obtain an immobilized cell aggregate. After the cultivation, the culture medium was removed and the immobilized cell aggregate was obtained. To the immobilized cell aggregate, an SD medium with 15mM acetanilide that is a substrate of the P450 was added and subjected to cultivation for 8 hours. This was the first cycle. This culture was repeated 5 times. The results are shown in Figure 1.

In Figure 1, the symbols ■, Δ , ○, and ● represent the concentration of glucose (g/L) , the concentration of free cells(g/L), the concentration of immobilized cells(mg/BS) and the concentration of acetoaminophen (µM), respectively.

In Table 1, the amount of P450 expressed in the immobilized yeast and free (not immobilized) yeast after the fifth cycle of the reaction is shown.

**Table 1**

| | Free yeast | Immobilized yeast |
|---|---|---|
| P450 (heme protein) (mole/cell) | not detected | 3.16×10⁻⁹ |
| P450 (heme protein) (molecule number /cell) | not detected | 19.02×10⁴ |

These results show that even when the immobilized yeast (immobilized cell aggregate) was repeatedly used, the conversion activity of acetanilide into acetaminophen (p-AAP) (i.e, the expression of p450) was not decreased at all. Therefore, it is demonstrated that a high expression of P450 was maintained.

As is shown in Table 1, the immobilized yeast expressed a large amount of P450. However, the free yeast expressed P450 with a very low level. It was confirmed that there was some yeast with lowered cell activity and some yeast which was dead in the free yeast.

As explained, the immobilized cell aggregate of the present invention comprises a support and cohesive or adhesive cells that are immobilized on the support. The cohesive or adhesive cells are capable of growth in the aggregate, and a cell of lowered cell activity or a dead cell can drop out from the immobilized cell aggregate due to loss of cohesive or adhesive activity. Therefore, only living cells can be immobilized, and thus the desired activity can be maintained. In view of the characteristics of the present invention, the immobilized cell aggregate of the present invention can be effectively used for production of a desired product, removal of pollutants and so on.

Various other modifications will be apparent to and can be readily made by those skilled in the art without departing from the scope and spirit of this invention. Accordingly, it is not intended that the scope of the claims appended hereto be limited to the description as set forth herein, but rather that the claims be broadly construed.

The present invention provides an immobilized cell aggregate. A cell with lowered cell activity or dead cell in the immobilized cell aggregate can drop out from the immobilized cell aggregate due to loss of cohesive or adhesive activity. Therefore, only living cells can remain in the immobilized cell aggregate. By using the immobilized cell aggregate, a desired material can be efficiently produced and environmental pollutants can be efficiently decomposed. The cohesiveness or adhesiveness can be obtained by incorporating genes. Moreover, by introducing genes for secretion and production for a desired material into cells of immobilized cell aggregate, the desired material can be efficiently produced.

## Claims

1. An immobilized cell aggregate comprising a support and cohesive or adhesive cells,
wherein a cohesive or adhesive cell can be immobilized on the support;
wherein the cohesive or adhesive cell is capable of growth in the aggregate; and
wherein a cell with lowered cell activity or a dead cell can drop out from the aggregate due to loss of its cohesive or adhesive activity.

2. The immobilized cell aggregate according to claim 1, wherein the cell is a non-cohesive or non-adhesive cell into which genes for cohesiveness or adhesiveness are introduced.

3. The immobilized cell aggregate according to claim 1, wherein the cell is cohesive yeast, a cohesive bacterium or an animal cell.

4. The immobilized cell aggregate according to claim 1, wherein the cell is cohesive yeast or non-cohesive yeast into which genes for cohesiveness are introduced.

5. The immobilized cell aggregate according to claim 1, wherein the support is a porous support.

6. The immobilized cell aggregate according to claim 1, wherein the aggregate is packed in a column.

7. A method for producing a desired material comprising the steps of subjecting an immobilized cell aggregate to cultivation,
wherein a cohesive or adhesive cell can be immobilized on the support;
wherein the cohesive or adhesive cell is capable of growth in the aggregate and can produce the desired material; and
wherein a cell with lowered cell activity or a dead cell can drop out from the aggregate due to loss of its cohesive or adhesive activity.

8. The method according to claim 7, wherein the cell is a non-cohesive or non-adhesive cell into which genes for cohesiveness or adhesiveness are introduced.

9. The method according to claim 7, wherein the desired material is secreted from the cell.

10. The method according to claim 9, wherein the desired material is secreted from the cell into which genes for secretion and production of the desired material are introduced.

11. The method according to claim 7, wherein the cell is cohesive yeast, a cohesive bacterium or an animal cell.

12. The method according to claim 7, wherein the cell is cohesive yeast or non-cohesive yeast into which genes for cohesiveness are introduced.

13. The method according to claim 7, wherein the support is a porous support.

14. The method according to claim 7, wherein the aggregate is packed in a column.

15. A method for selecting a cohesive or adhesive cell that can: attach to a support so as to form an immobilized cell aggregate; grow in the immobilized cell aggregate; produce a desired material; and drop out from the immobilized cell aggregate due to a lowered cell activity or death;
wherein the method comprises:
introducing genes for cohesiveness or adhesiveness and/or genes for producing the desired material into a cell so as to obtain a recombinant cell;
cultivating the recombinant cell with the support so as to form an immobilized cell aggregate;
detecting and/or measuring the desired material that is produced from the immobilized cell aggregate;
and recovering the cohesive or adhesive cell.

16. A method for preparing an immobilized cell aggregate comprising a support and cohesive or adhesive cells, wherein a cohesive or adhesive cell is capable of growth in the aggregate and wherein a cell with lowered cell activity or a dead cell can drop out from the aggregate due to loss of its cohesive or adhesive activity;
wherein the method comprises:
introducing genes for cohesiveness or adhesiveness and/or genes for producing the desired material into a cell so as to obtain a recombinant cell;
cultivating the recombinant cell with the support so as to form an immobilized cell aggregate; and recovering the immobilized cell aggregate.

17. An expression cassette which comprises genes for cohesiveness or adhesiveness and genes for regulating the production of a desired material,
wherein the cassette is expressed so as to convert a non-cohesive or non-adhesive cell into a cohesive or adhesive cell; the cohesive or adhesive cell being immobilized on a support so as to form an immobilized cell aggregate; the cohesive or adhesive cell being capable of growth in the aggregate; a cell of lowered cell activity or a dead cell being able to drop out from the support due to loss of cohesive or adhesive activity; and
Wherein the cassette is expressed so as to produce the desired material.

18. The cassette according to claim 17, wherein the cassette has genes for cohesiveness of yeast and a promoter sequence for regulating the expression of the gene for the desired material of the yeast.
